# EUROPÄISCHE PATENTSCHRIFT

(11) **EP 2 014 312 B1**
(45) Veröffentlichungstag und Bekanntmachung des Hinweises auf die Patenterteilung: **08.05.2013**
(21) Anmeldenummer: 08016545.9
(22) Anmeldetag: 02.12.2002
(51) Int. Cl.: A61L 2/28, A61L 2/18, A61L 2/24

(54) **Reinigungs- und Desinfektionsmaschine**
Cleaning and disinfecting machine
Machine à nettoyer et désinfecter

(30) Priorität: 12.12.2001 DE 10161106
(43) Veröffentlichungstag der Anmeldung: 14.01.2009
(62) Teilanmeldung aus: 02026778.7
(73) Patentinhaber: BHT Hygienetechnik GmbH, 86368 Gersthofen (DE)
(72) Erfinder: Annecke, Karl Heinz, Dr., 64625 Bensheim (DE)
(74) Vertreter: von Bülow, Tam

(56) Entgegenhaltungen:
- EP-A- 1 000 629
- EP-A- 1 130 381
- US-A- 6 086 822
- US-A1- 2001 033 805

## Beschreibung

Die Erfindung bezieht sich auf eine Reinigungs- und Desinfektionsmaschine gemäß dem Oberbegriff des Patentanspruches. Solche Maschinen sind allgemein bekannt und werden von der Anmelderin seit Jahren vertrieben.

Die EP 1 000 629 A1 zeigt einen Sterilisator mit einem Messwertaufnehmer zur Ermittlung einer Zustandsgröße eines Prozessmediums des Sterilisators und mit einem Referenzmesswertaufnehmer. Als Messwertaufnehmer kann es sich um einen Drucksensor, einen Temperatursensor, einen Messwertaufnehmer für Feuchte, Konzentrationen oder Inertgas Anteile im Dampf sowie um Sensoren zur Erfassung eines Gas-Volumenstromes oder einen Messwertaufnehmer zur Beurteilung des Dampfzustandes handeln. Als Referenzmesswertaufnehmer für dieselben Größen können bauartidentische Messwertaufnehmer verwendet werden oder ein Messwertaufnehmer, der wenigstens zwei Referenzmesswerte mit besonders hoher Genauigkeit liefert. Das genannte Prozessmedium des Sterilisators ist üblicherweise überhitzter Wasserdampf. Die Referenzmesswertaufnehmer werden lediglich bei einem Abgleichvorgang eingesetzt und dienen dann dazu, die Genauigkeit der für den Normalbetrieb vorgesehenen Messwertaufnehmer zu überprüfen.

Die EP 1 130 381 A1 beschreibt eine Analysevorrichtung für die Messung des Reinheitsgrades von Wasser. Die Vorrichtung hat eine Messkammer mit einem Einlass und einem Auslass. In der Kammer sind mehrere optische Sensoren angeordnet, die den Transmissionsgrad des Wassers messen. Zusätzlich kann dem Einlass ein Durchflussmesser vorgeschaltet sein. Die drei Sensoren für die Messung des Transmissionsgrades arbeiten nach demselben physikalischen Messprinzip.

Die US 2001/0033805 A1 beschreibt ein Reinigungsverfahren für medizinische Geräte mit einer Reinigungskammer, die einen Einlass und einen Auslass hat und daher von einer Reinigungsflüssigkeit durchströmt werden kann. Die Schrift schlägt eine Vielzahl möglicher Sensoren vor, mit denen Verunreinigungen, wie z.B. organische Verunreinigungen, anorganische Verunreinigungen etc., des die Vorrichtung durchfließenden Reinigungsmittels gemessen werden können, so dass in einigen Ausführungsbeispielen auch die Anwendung mehrerer unterschiedlicher Sensoren vorgeschlagen wird, mit denen Proteine und andere organische Substanzen sowie anorganische Substanzen detektiert werden können.

Im Zusammenhang mit der Durchsetzung des Medizinproduktegesetzes und den daraus entstehenden Ausführungsverordnungen und Normen für Reinigungs- und Desinfektionsmaschinen wird das Thema "Validierung des Reinigungs-/Desinfektionsverfahrens" bzw. Validierung der Reinigung und Desinfektion gefahrener Parameter zunehmend wichtiger. Validierung heißt, dass eine physikalische Größe (z.B. Volumen, Temperatur, Zeit oder ähnliches) auf der einen Seite durch eine Steuerung und dazu notwendige Sensoren so eingestellt wird, dass die an den Wasch- und Desinfektionsprozess gestellten Anforderungen erfüllt werden. Andererseits muss aber durch einen davon unabhängigen Sensor bzw. ein davon unabhängiges Messverfahren zwecks Validierung bzw. dann auch nachfolgender Dokumentation festgestellt werden, ob die der Steuerung eingegebenen Sollwerte tatsächlich während des Wasch- und Desinfektionsprozesses eingehalten wurden. Dies heißt beispielsweise bei der Desinfektionstemperatur, dass die Steuerung, die die Heizung zum Erreichen der notwendigen Desinfektionstemperatur ein- und ausschaltet, auf der einen Seite einen Temperaturfühler besitzt und dass auf der anderen Seite ein ähnlich oder gleicher Temperaturfühler in der Kammer angebracht ist, der überprüft, ob die vorgegebene Temperatur tatsächlich erreicht wurde und der dieses beispielsweise in einem Protokollausdruck niederlegt.

Nachteil bei der Verwendung von in diesem Falle zwei gleichartigen Temperaturfühlern und eventuell auch gleichen Strecken in der Auswertung (z.B. Analog/Digitalwandler; Mikrocontroller usw.) ist, dass sich z.B. Serienfehler in den Temperaturfühlern oder auch Verarbeitungsfehler auf der weiteren Verarbeitungsstrecke dahingehend auswirken, dass die Steuerung auf der einen Seite die verkehrte Temperatur einstellt und der Validierungsfühler auf der anderen Seite aber nicht feststellt, dass die Solltemperatur nicht erreicht wurde, da er ebenfalls die Auswertung mit dem gleichen Fehler behaftet vornimmt.

Aufgabe der Erfindung ist es daher, die bekannte Reinigungs- und Desinfektionsmaschine dahingehend zu verbessern, dass eine höhere Sicherheit der Validierung erreicht wird.

Diese Aufgabe wird durch die im Patentanspruch angegebenen Merkmale gelöst.

Grundprinzip der Erfindung ist es, für die jeweils zu erfassende Messung der Menge einer Flüssigkeit zwei Messfühler zu verwenden, die auf unterschiedlichen physikalischen Messverfahren beruhen. Diese physikalischen Messprinzipien sind einerseits ein Durchflussmesser und andererseits ein Drucksensor, der in Zusammenwirken mit Öffnungszeiten eines Ventils aus dem gemessenen Wasserdruck und bekannten Parametern, wie Leitungsquerschnitte und Strömungswiderstand, in Verbindung mit einer Steuereinheit die geförderte Menge der Flüssigkeit misst.

Damit können sich die beiden Meßmethoden gegenseitig kontrollieren und, da beide unterschiedlichen physikalischen Messverfahren unabhängig voneinander sind, Fehler des einen Messverfahrens erkannt werden. Würde man beispielsweise lediglich zwei hintereinander geschaltete Durchflussmesser verwenden, die beide also auf dem gleichen physikalischen Messprinzip basieren, so würden systematische Fertigungsfehler der Durchflussmesser oder Auswertefehler der Impulse von beiden Durchflussmessern ein falsches Signal liefern und der Prozess wäre nicht sicher validierbar.

Wie bekannt, spielt in der Reinigungstechnik nicht nur die absolute Menge des Reinigungs- bzw. Desinfektionsmittels eine Rolle, sondern auch die prozentuale Beimischung zum zugelassenen Wasser, so dass eine weitere physikalische Größe, die möglichst exakt ermittelt werden sollte, die zugeführte Wassermenge ist. Auch hier gibt es wieder verschiedene Möglichkeiten, die zugeführte Wassermenge durch zwei unterschiedliche physikalische Parameter zu erfassen.

Im folgenden wird die Erfindung anhand eines Ausführungsbeispieles im Zusammenhang mit der Zeichnung ausführlicher erläutert.

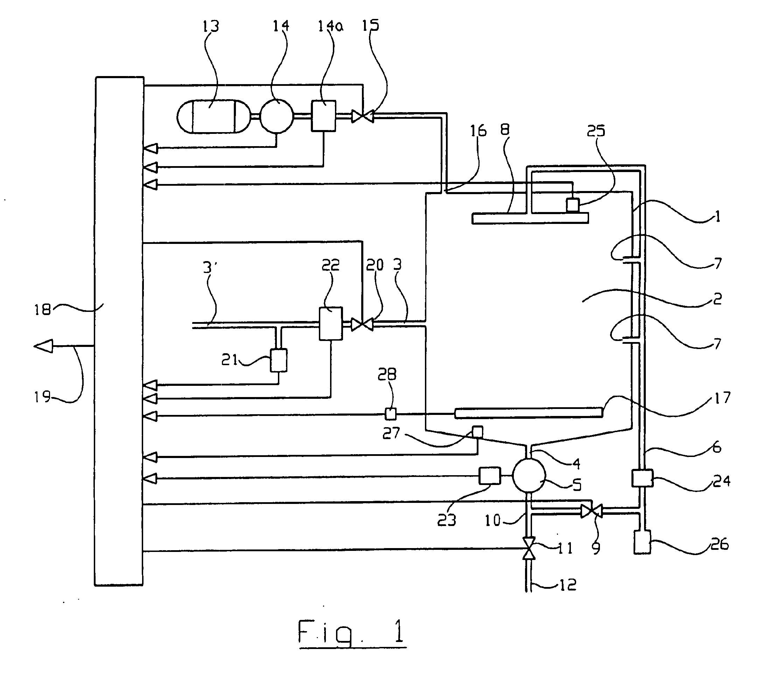

Die einzige Figur 1 zeigt ein Prinzipschaltbild einer Reinigungs- und Desinfektionsmaschine nach der Erfindung.

Die Reinigungs- und Desinfektionsmaschine, im folgenden "Waschmaschine 1" genannt, hat einen Waschraum 2 mit einem Frischwassereinlass 3, einem Auslas 4, einem Umwälzkreislauf mit einer Umwälzpumpe 5, einer Umwälzleitung 6, die über ein oder mehrere Düsen 7 und/oder einen oder mehrere rotierende Wascharme 8 in den Waschraum 2 mündet, wobei in der Umwälzleitung ein elektrisch steuerbares Ventil 9 vorgesehen ist. An den Ausgang der Umwälzpumpe 5 ist ein Verzweigungsstück 10 angeschlossen, das einerseits mit der Umwälzleitung 6 und andererseits über ein Ventil 11 mit einem Abwasserkanal 12 verbunden ist.

Weiter ist ein Vorratsbehälter 13 für Reinigungs- und/oder Desinfektionsmittel vorgesehen, der über eine Dosierpumpe 14 und ein Ventil 15 mit einem Einlass 16 in den Waschraum 2 der Maschine verbunden ist.

Im Inneren des Waschraumes ist eine Heizeinrichtung 17 angeordnet, die üblicherweise elektrisch betrieben wird.

Der gesamte Reinigungs- und Desinfektionsvorgang wird von einer Steuereinheit 18 gesteuert und überwacht, die an einem Ausgang 19 auch Protokolldaten über sämtliche Abläufe ausgeben kann, womit das Reinigungs- und Desinfektionsergebnis validiert wird.

Insoweit handelt es sich bisher um ein Beispiel einer herkömmlichen Waschmaschine.

Um ein einwandfreies, reproduzierbares und nachweisbares Reinigungs- und Desinfektionsergebnis erzielen zu können, müssen sämtliche Arbeitsabläufe überwacht und dokumentiert werden. Hierzu sind eine Vielzahl von Messfühlern vorgesehen, mit denen diverse Parameter gemessen werden und von der Steuerung ausgewertet werden. Beispielsweise sind folgende Parameter zu überwachen:
- Frischwassermenge
- Menge des Reinigungs- und Desinfektionsmittels
- Menge des umgewälzten aktuellen Waschwassers
- Temperatur des Waschwassers
- Menge des Abwassers.

Alle bekannten Sensoren für diese Größen können fehlerhafte Messergebnisse liefern. Verwendet man zur Überprüfung der Messergebnisse jeweils einen weiteren gleichartigen Sensor, so können sich z.B. Serienfehler der Sensoren oder auch Verarbeitungsfehler dahingehend auswirken, dass die Steuerung auf der einen Seite einen verkehrten Wert einstellt und der zweite Sensor, der als Validierungsfühler dient, auf der anderen Seite aber nicht feststellt, dass der Sollwert nicht erreicht wurde, da dessen Auswertung mit dem gleichen Fehler behaftet ist. Der wesentliche Kerngedanke der Erfindung liegt darin, für die Regelung bzw. Steuerung des jeweiligen Parameters und für dessen Überprüfung bzw. Validierung jeweils Messfühler zu verwenden, die auf einem unterschiedlichen physikalischen Messprinzip basieren. Dies wird nun im folgenden anhand verschiedener Parameter erläutert.

### Frischwasserzufuhr

Die Frischwasserzufuhr von dem Frischwassereinlass 3' wird über ein Ventil 20 gesteuert. Anhand der Öffnungszeit des Ventils, dem von einem Drucksensor 21 gemessenen Wasserdruck und bekannten Parametern, wie Leitungsquerschnitte, Strömungswiderstand etc. lässt sich die zugeführte Frischwassermenge ermitteln. Als ein auf anderem physikalischen Messprinzip basierender Sensor ist ein Durchflussmesser 22 vorgesehen, der in der Zuleitung zum Frischwassereinlass 3 angeordnet ist und die Wassermenge misst. Die beiden aufgrund unterschiedlicher physikalischer Messmethoden ermittelten Messwerte können verglichen werden, so dass sich beide Sensoren wechselseitig überwachen.

### Dosierung von Reinigungs- und Desinfektionsmitteln nicht Gegenstand der Erfindung)

Als erster Sensor dient hier die Dosierpumpe 14, deren Umdrehungszahl proportional der geförderten Menge ist. Wird für den Antrieb beispielsweise ein Motor mit Umdrehungs- bzw. Drehwinkelkontrolle verwendet, so ist die Anzahl der Umdrehungen oder die Laufzeit des Motors ein genaues Maß für die geförderte Menge. Als zweiter Sensor ist hier ein Durchflussmesser 14a vorgesehen, der in der Zuleitung von dem Vorratsbehälter 13 zum Einlass 16 angeordnet ist. Auch hier wird wieder die durchgeflossene Menge mit Sensoren ermittelt, die auf unterschiedlichen physikalischen Meßprinzipien beruhen.

### Menge des umgewälzten Wassers

Hier können an die Umwälzleitung 6 ein Drucksensor 26 und ein Durchflussmesser 24 angeschlossen sein. Aus den Messwerten des Drucksensors 26 kann zusammen mit der Erfassung der Öffnungs- und Schließzeiten des Ventils 9 und den bekannten Leitungsparametern wiederum die umgewälzte Wassermenge ermittelt werden.

Der in Fig. 1 dargestellte Sensor 23, der beispielsweise die Umdrehungszahl der Umwälzpumpe 5 misst, ist nicht Gegenstand der Erfindung.

Auch die Messung der Temperatur des aktuellen Waschwassers, die mit einem Temperaturfühler 27 vorgenommen wird, ist nicht Gegenstand der Erfindung. Gleiches gilt für den in Fig. 1 dargestellten Sensor 28, der den von der Heizeinrichtung 17 aufgenommenen Strom misst, woraus sich in Kenntnis der im Waschraum befindlichen Wassermenge die Temperatur ermitteln lässt.

Auch hier kann, was nicht Gegenstand der Erfindung ist, die Förderleistung der Umwälzpumpe 5 durch einen Sensor 23 gemessen werden, der beispielsweise die Umdrehungszahl misst, was bei ansonsten bekannten Parametern, wie Leitungsquerschnitte etc. ein Maß für die umgewälzte Menge ist. Der andere Sensor kann wiederum ein Durchflussmesser 24 sein.

Alternativ oder kumulativ kann, was nicht Gegenstand der Erfindung ist, auch ein Drehsensor 25 verwendet werden, der die Umdrehungen des Dreharmes 8 misst. Bei bekannten Leitungsparametern ist nämlich die Drehzahl des Dreharmes 8 wiederum ein Maß für die in der Umwälzleitung 6 umgewälzte Wassermenge.

### Temperatur, nicht Gegenstand der Erfindung

Für die Messung der Temperatur des aktuellen Waschwassers ist ein Temperaturfühler 27 vorgesehen. Der zweite Messwert kann durch Erfassung der Heizleistung der Heizeinrichtung 17 ermittelt werden, beispielsweise durch einen Sensor 28, der den von der Heizeinrichtung 17 aufgenommenen Strom misst. In Kenntnis der im Waschraum befindlichen Wassermenge lässt sich hierüber die Temperatur ermitteln und mit dem Messwert des Sensors 27 vergleichen.

## Patentansprüche

1. Reinigungs- und Desinfektionsmaschine, mit einem Waschraum (2), dem über ein von einer Steuereinheit (18) angesteuertes Ventil (9, 20) eine Flüssigkeit zugeführt oder in dem Waschraum (2) umgewälzt wird, mit mindestens einem Sensor, der die Menge der Flüssigkeit misst,
**dadurch gekennzeichnet,**
**dass** zur Messung der Menge der Flüssigkeit zwei Sensoren (24, 26; 22, 20) verwendet werden, die je mit unterschiedlichen physikalischen Messprinzipien arbeiten,
wobei der eine Sensor ein Durchflussmesser (22, 24) ist und der andere Sensor ein Drucksensor (21, 26), der in Zusammenwirken mit Öffnungszeiten des Ventils (20, 9) aus dem gemessenen Wasserdruck und bekannten Leitungsparametern in Verbindung mit der Steuereinheit (18) die geförderte Menge der Flüssigkeit misst.

## Claims

1. Cleaning and disinfecting machine, having a washing chamber (2), to which a liquid is supplied via a valve (9, 20) contolled by a control unit (18) or is circulated in the washing chamber (2), with at least one sensor which measures the amount of the liquid,
**characterized in that**,
for measuring the amount of the fluid two sensors (24, 26, 22, 20) are used, which work with different physical measuring principles,
wherein the one sensor is a flow meter (22, 24) and the other sensor is a pressure sensor (21, 26) which in interaction with opening times of the valve (20, 9), with the measured water pressure and with known parameters of fluid lines, is measuring the delivered amount of the liquid in conjunction with the control unit (18).

## Revendications

1. Machine de nettoyage et de désinfection, avec une chambre de lavage (2), à qui un liquide est fourni par un soupape à commande (9, 20) contrôlé par une unité de commande (18), ou est mis en circulation dans la chambre de lavage (2),
avec au moins un capteur qui mesure la quantité du liquide,
**caractérisé en ce**
**que**, pour mesurer la quantité du fluide on utilise deux capteurs (24, 26, 22, 20), qui fonctionnent selon des principes de mesure physiques différents, parmi lesquelles
un capteur est un débitmètre (22, 24) et l'autre capteur est un capteur de pression (21, 26) qui, en interaction avec les temps d'ouverture de la soupape (20, 9) à partir de la pression de l'eau mesurée et des paramètres connus de ligne du liquide en liaison avec l'unité de commande (18), mesure le montant de la livraison de liquide fourni.
